# FASCICULE DE BREVET EUROPEEN

(11) **EP 1 131 615 B1**
(45) Date de publication et mention de la délivrance du brevet: **26.12.2007**
(21) Numéro de dépôt: 00962639.1
(22) Date de dépôt: 14.09.2000
(51) Int. Cl.: G01N 1/24, E21B 21/06, E21B 49/00, G01N 33/28

(54) **METHODE ET SYSTEME D'EXTRACTION, D'ANALYSE ET DE MESURE SUR DES CONSTITUANTS TRANSPORTES PAR UN FLUIDE DE FORAGE**
VERFAHREN UND VORRICHTUNG ZUR EXTRAKTION, ANALYSE UND MESSUNG VON IN EINER BOHRFÜSSIGKEIT MITGEFÜHRTEN KOMPONENTEN
METHOD AND SYSTEM FOR EXTRACTING, ANALYSING AND MEASURING CONSTITUENTS TRANSPORTED BY A BORE FLUID

(30) Priorité: 24.09.1999 FR 9912032
(43) Date de publication de la demande: 12.09.2001
(73) Titulaire: Institut Français du Pétrole, 92852 Rueil-Malmaison Cedex (FR); Geoservices S.A, 93151 Le Blanc-Mesnil Cedex (FR)
(72) Inventeur: DURIEZ, Gilbert, F-92500 Rueil-Malmaison (FR); LECANN, Jean-Paul, F-77230 Dammartin EN Goële (FR); BREVIERE, Jérôme, F-95350 Saint Brice sous Forêt (FR); DE HEMPTINNE, Jean-Charles, F-78110 Le Vésinet (FR)
(86) Numéro de dépôt international: PCT/FR2000/002543
(87) Numéro de publication internationale: WO 2001/022050

(56) Documents cités:
- EP-A- 0 370 548
- FR-A- 2 646 508
- US-A- 4 250 142
- US-A- 5 499 531
- US-A- 5 663 492
- US-A- 5 693 538

## Description

La présente invention concerne une méthode et un système pour l'analyse et la mesure de constituants liquides ou gazeux contenus dans un fluide de forage. Par constituants, on désigne ici des hydrocarbures, par exemple de C1 à C8 incluant benzène, toluène, xylène, ou des gaz tels que H2S, CO2, O2, H2, N2. Ces constituants proviennent de l'opération de forage à travers des couches de terrain, opération qui a pour effet de briser la roche en libérant les gaz ou les fluides contenus dans les pores de roche. Le forage se fait conventionnellement avec une circulation d'un fluide, dit de forage, qui a, entre autres, pour fonction de nettoyer l'outil de forage et de remonter les débris de roche à la surface du sol. Les constituants en question remontent donc également par entraînement vers la surface par l'intermédiaire de ce vecteur. Il est clair que, compte tenu du débit de fluide de forage comparé à la vitesse de destruction de la roche, la quantité volumique desdits constituants est toujours relativement faible comparée au volume de boue.

On connaît des installations permettant d'effectuer des mesures qualitatives et quantitatives sur du gaz C1-C5 contenu dans un fluide de forage, mesures (ou diagraphies) qui permettent d'identifier les zones géologiques forées pour des raisons de sécurité du forage et/ou du personnel. Le document FR 2646508 décrit un procédé et un appareil pour prélever en continu des échantillons gazeux contenus dans un liquide chargé de solides, notamment un fluide de forage. Dans ce document, ni son mode de dégazage, ni son mode de transport, ne permet pas d'extraire et de transporter les éventuels hydrocarbures présents sous forme liquide dans la boue de retour à la surface du sol.

Le document US 5663492 décrit un dispositif et une méthode pour l'analyse en continu et/ou la modification des caractéristiques de pression de vapeur d'un flux hydrocarboné liquide, comprenant une séparation gaz-liquide, des moyens d'entrée du flux liquide, des moyens de sortie de liquide pour le maintien du niveau de liquide dans la chambre de séparation, des moyens de contrôle de la température, des moyens de sortie du gaz, des moyens de contrôle de la pression et des moyens d'analyse métrologique des différents flux.

Le document US 5693538 décrit un système et une méthode pour le monitorage des espèces volatiles dans les liquides dans lesquels le liquide est injecté dans une chambre de vaporisation.

Le document US 5499531 décrit un système et une méthode de détermination des constituants volatiles et de la pression de vapeur de liquides de composition inconnue comprenant des moyens de séparation gaz-liquide, des moyens de mesure de la composition gazeuse par chromatographie et des moyens de calcul permettant la modélisation de la composition du liquide.

Le document EP 0370548 décrit un système et une méthode pour l'analyse quantitative de gaz produits par un puits foré dans lesquels l'étape de capture des gaz libérés comprend l'abaissement de la pression à l'intérieur du tube fontaine jusqu'à, une valeur inférieure au tube fontaine, et les gaz libérés et les gaz extraits sont analysés ensemble au niveau des moyens d'analyse de gaz.

La présente invention a pour objet de mettre en oeuvre des conditions nécessaires: pour l'extraction de constituants gazeux ou liquides contenus dans un fluide de forage, pour transporter ces constituants sous une forme gazeuse, et pour effectuer des analyses et des mesures sur ces constituants. Pour effectuer des analyses correctes, permettant de mieux déterminer la nature et la composition des terrains traversés par un forage, les constituants ne doivent pas condenser dans les éléments du système et le temps de transit de ces constituants entre le point d'extraction et le point de mesure doit être acceptable pour permettre une surveillance de l'opération de forage.

Ainsi, la présente invention concerne un système d'extraction, d'analyse et de mesure sur des constituants entraînés par un fluide de puits au cours d'opérations de forage. Le système comporte en combinaison:
des moyens de prélèvement d'un volume déterminé du fluide,
des moyens d'extraction sous forme vapeur des constituants contenus dans le volume de fluide comportant un espace en dépression,
une conduite de transport des vapeurs dont une première extrémité communique avec l'espace, la seconde extrémité étant reliée à une pompe à dépression,
des moyens d'échantillonnage placés dans le voisinage de la seconde extrémité comportant une boucle d'échantillonnage qui permet le prélèvement d'une quantité déterminée des vapeurs circulant dans ladite conduite,
des moyens de distribution qui injectent la quantité dans des moyens d'analyse et de mesure, et
des moyens de contrôle de la température des moyens d'échantillonnage afin d'éviter toute condensation de la quantité de vapeurs.

L'espace qui contient le fluide de forage prélevé peut être étanche vis-à-vis de l'ambiance 'extérieur.

L'espace peut comporter un orifice d'entrée d'un gaz auxiliaire, par exemple de l'air ou de l'azote.

Les moyens d'extraction peuvent comporter des moyens de chauffage dudit volume de fluide prélevé.

La boucle d'échantillonnage peut être montée directement en parallèle sur la ligne de transport.

Les moyens de prélèvement du fluide de forage peuvent comprendre une pompe d'aspiration et/ou une pompe de refoulement.

L'espace peut être à une pression absolue comprise entre 10 et 100 mb et l'aspiration de la pompe à dépression peut être comprise entre 1 et 10 mb.

L'invention concerne également une méthode d'extraction, d'analyse et de mesure sur des constituants entraînés par un fluide de puits au cours d'opération de forage, dans laquelle on effectue les étapes suivantes:
on prélève un volume de fluide de forage pour le placer dans un espace faisant partie de moyens d'extraction des constituants sous forme vapeur,
on applique une dépression dans l'espace par l'intermédiaire d'une conduite reliant les moyens d'analyse et de mesure aux moyens d'extraction,
on prélève dans le voisinage des moyens d'analyse et de mesure une quantité déterminée des vapeurs circulant dans la conduite,
on injecte la quantité de vapeurs dans les moyens d'analyse et de mesure,
on déduit la nature et/ou la quantité des constituants qui ont été vaporisés dans les moyens d'extraction.

Selon la méthode, on peut ajuster les conditions de dépression et/ou de température pour l'extraction sous forme vapeur en fonction des paramètres de forage, par exemple nature du fluide, débit, température ambiante, temps de transit dans la conduite.

On peut régler un débit d'entrée de gaz auxiliaire dans l'espace.

On peut appliquer une dépression comprise entre 10 et 100 mb absolu dans l'espace d'extraction.

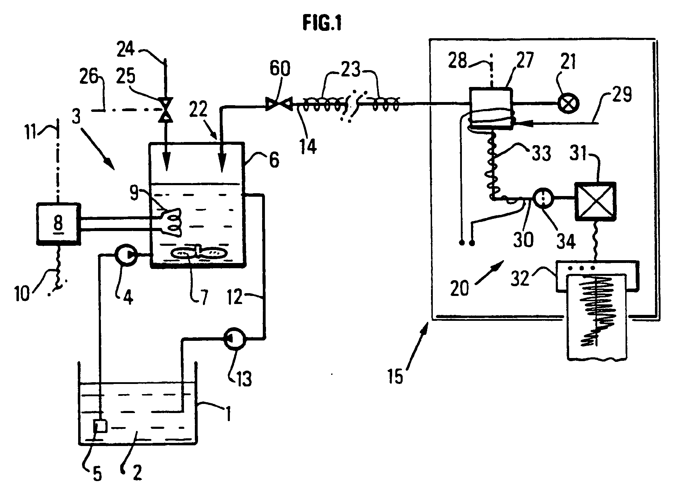

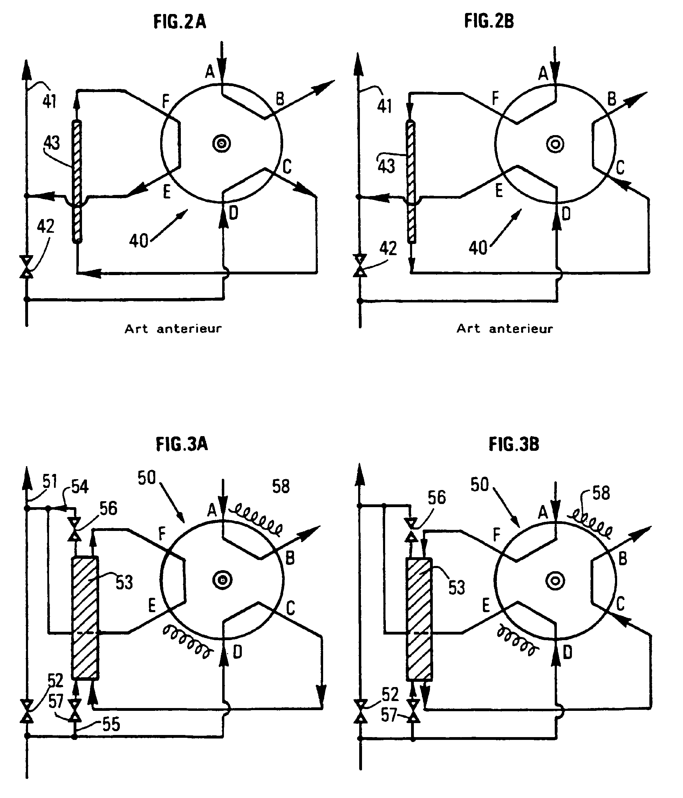

La présente invention sera mieux comprise et ses avantages apparaîtront plus clairement à la lecture de la description qui suit d'un exemple de réalisation, nullement limitatif, illustré par les figures ci-annexées, parmi lesquelles:
■ la figure 1 représente un schéma de l'ensemble du système selon l'invention,
■ les figures 2a et 2b montrent le dispositif d'échantillonnage, selon l'art antérieur, pour effectuer l'analyse et la mesure des constituants dans un chromatographe,
■ les figures 3a et 3b montrent le dispositif d'échantillonnage, selon l'invention, pour effectuer l'analyse et la mesure des constituants dans un chromatographe dans les conditions présentes.

Sur la figure 1, la référence 1 désigne une goulotte dans laquelle s'écoule le fluide de forage 2 de retour du fond du puits par circulation dans l'annulaire défini par la garniture de forage et le puits foré. Cette goulotte 1 conduit le fluide de forage de la tête de puits à l'installation de surface pour le traitement et la séparation des solides. Les moyens d'extraction 3 des constituants à analyser comportent de préférence une pompe 4 dont l'aspiration 5 plonge dans le fluide de forage et dont le refoulement débouche dans un récipient 6 dont le volume permet de garder en tampon un volume de fluide déterminé pendant un temps donné, lequel temps est fonction de ce volume et du débit de la pompe 4. Le volume de fluide de forage contenu dans le récipient 6 est soumis à une agitation pour favoriser l'extraction par dégazage et évaporation. Cette agitation peut être mécanique, par exemple, par le moyen d'agitateur rotatif 7, ou de moyens ultrasonores.

Pour obtenir les conditions d'extraction désirées, des moyens de chauffage 8 peuvent comporter une canne chauffante 9 à l'intérieur du récipient 6, une alimentation électrique 10 et une ligne de commande et de régulation 11. D'autres moyens de chauffage du volume de fluide dans le récipient peuvent être utilisés, par exemple par l'extérieur ou une double paroi chauffante. Le récipient comprend également une évacuation de la boue 12 pour le retour à la goulotte du débit de fluide prélevé par la pompe. Il est envisageable de placer une pompe 13 sur cette ligne de refoulement de façon à mieux contrôler le débit de circulation du fluide de forage en transit dans le récipient 6.

Une conduite 14 relie les moyens d'extraction 3 aux moyens d'analyse et de mesure 20. La longueur de cette conduite peut être comprise entre 50 et 200 m car les moyens de mesure 20 sont placés dans une cabine (symbolisée par le cadre 15) placée à une distance suffisante de la tête de puits pour être dans une zone sécurisée. A l'extrémité de cette conduite, une pompe à vide 21 crée une dépression qui peut atteindre quelques millibars (par exemple entre 1 et 10 mb). Compte tenu des pertes de charge et du débit de gaz en circulation, la dépression à l'entrée 22 de la conduite peut varier, par exemple entre 10 et 100 mb. Des moyens de contrôle de la dépression 60 peuvent être placés dans le voisinage de l'extrémité de la ligne 14, coté moyens d'extraction 3. Ces moyens peuvent être une vanne, une duse, ou tout autres moyens pouvant faire varier la dépression dans la ligne et/ou dans le récipient 6. Le diamètre intérieur de la conduite de transport 14 est compris entre 5 et 15 mm, de préférence environ 10 mm. Le matériau du tube intérieur est choisi pour être imperméable aux hydrocarbures comme par exemple les tuyaux d'alimentation en carburant. Pour éviter la condensation des gaz transportés, dans certaines situations extrêmes, il peut être indispensable de chauffer au moins une portion de la conduite, par exemple par un cordon chauffant 23.

Le récipient 6 comporte une entrée 24 d'un gaz, air ou gaz inerte, contrôlée par une vanne 25 permettant de régler le débit d'entrée de gaz. Cette vanne peut être pilotée à l'aide d'une ligne 26.

En série sur la conduite 14, des moyens d'échantillonnage 27 sur le flux de gaz transporté prélèvent une certaine quantité de gaz selon un rythme dépendant notamment du cycle de mesure. L'échantillonneur est piloté par la ligne 28. Les moyens d'échantillonnage 27 comprennent: une boucle dite "de chromatographe" dans laquelle un volume de gaz est prélevé de la conduite 14, des vannes ou distributeurs pour effectuer ce prélèvement puis faire pénétrer ce volume de gaz en le poussant par un fluide inerte. Ces différents constituants seront plus précisément détaillés à l'aide des figures 3a et 3b. La figure 1 montre schématiquement: la conduite d'injection 29 d'un gaz vecteur destiné au déplacement de l'échantillon de gaz prélevé sur la conduite 14 par les moyens d'échantillonnage 27, la conduite d'amenée 30 de ce volume de gaz à l'analyseur 31, par exemple un appareil de chromatographie. Les mesures effectuées par l'appareil 31 sont envoyés par l'intermédiaire d'une interface électronique à une unité centrale d'ordinateur qui traite ces mesures pour fournir, par exemple un enregistrement donnant la nature et les quantités des constituants remontés avec le fluide de forage, en fonction de la profondeur du forage.

Au moins une zone des moyens d'échantillonnage 27 et une portion de la conduite 30 peuvent être chauffés par des moyens de chauffage 33, ceci afin d'éviter toute condensation avant ou pendant la mesure. On peut, de plus, placer sur cette conduite 30 des moyens d'élimination 34, ou au moins de diminution, de la teneur en vapeur d'eau du flux gazeux en circulation.

Les figures 2a et 2b décrivent une vanne d'échantillonnage 40 conventionnelle selon l'art antérieur, figure qui permet de mieux apprécier les améliorations importantes de la présente invention. Sur la figure 2a, la ligne 41 est en communication avec un dégazeur atmosphérique (non représenté), les gaz prélevés sur le fluide étant en déplacement dans la ligne 41 grâce à une circulation d'air. La vanne 42, une fois fermée ou partiellement fermée, dérive l'effluent de façon à traverser la vanne 40 par les voies D et C pour passer ensuite dans la boucle d'échantillonnage 43, avant de traverser à nouveau la vanne par les voies F et E pour rejoindre la ligne principale 41. La voie A est reliée à une alimentation d'un gaz vecteur (air, hélium, hydrogène ou azote). La voie B est reliée à l'analyseur chromatographique. La figure 2b montre le circuit en position analyse. La position du boisseau de la vanne 40 a été changée de façon que le gaz vecteur en A communique avec la voie F pour déplacer l'effluent piégé dans la boucle 43 vers l'analyseur en B en passant par la voie C. Une dérivation de la ligne 41 passe par les voies D et E. Le volume de gaz envoyé dans l'analyseur est environ de quelques dizaines de micro litre (10⁻⁶ l) sous une pression très proche de la pression atmosphérique.

La figure 3a montre une vanne d'échantillonnage 50, qui peut être similaire à la vanne 40 de l'art antérieur. La ligne 51 est en dépression puisque raccordée à une pompe à vide. Une boucle d'échantillonnage 53 peut être reliée à la ligne et à la vanne 50 de la même façon que dans l'art antérieur, mais de plus, des conduits 55 et 54 communiquent directement avec la ligne en vide partiel 51. Des vannes 56 et 57 commandées ouvrent ou ferment ces communications. La boucle d'échantillonnage 53 est d'un volume beaucoup plus important que celle de l'art antérieur puisque le volume de l'effluent est faible à la pression absolue. Dans la réalisation ici décrite le volume de la boucle 53 est d'environ 1000 µl. Sur la figure 3a, la vanne 50 est en position d'échantillonnage, l'effluent aspiré par la pompe à vide traverse la boucle 53 grâce aux dérivations directes 55 et 54, les vannes 56 et 57 étant ouvertes. La vanne 52 peut être plus ou moins fermée, en fonction de la perte de charge qu'il peut être nécessaire de créer. Accessoirement, la dérivation passant par C, D, F et E est toujours opérante. Ainsi, en choisissant un diamètre intérieur suffisant pour les lignes 55 et 54, les pertes de charges importantes dans la vanne dues au vide partiel nécessaire à la présente invention, sont minimisées. La figure 3b montre les circuits en position d'analyse. Les vannes 56 et 57 sont fermées, la vanne d'échantillonnage distribue le gaz vecteur de la voie A à la voie F pour pousser l'effluent contenu dans la boucle 53 vers l'analyseur chromatographique en B. De préférence, la vanne 52 est suffisamment ouverte de façon que la dépression ne soit pas limitée par les pertes de charge dans le circuit D et E. L'effluent contenu dans la boucle est partiellement comprimé par le gaz vecteur, ce qui impose un contrôle de la température de tous ces circuits, boucle et vannes, pour éviter la condensation des constituants de l'effluent. Un système de chauffage 58 maintient une température suffisante, par exemple environ 200°C.

Pour tester le système selon l'invention, les mesures suivantes ont été effectuées sur une maquette comprenant une ligne de 100 m ou 200 m ayant un diamètre intérieur de 10 mm. Une vanne de contrôle du débit d'air entrant est montée sur une première extrémité de cette ligne. La pression Pe est mesurée à cette extrémité. La pression Ps est mesurée à l'autre extrémité, laquelle est prolongée par un tube de 6 mm de diamètre intérieur sur 5,7 m pour être raccordé à une pompe à vide où la pression Pppe est également mesurée.

En fonction du débit d'air (mesuré à la pression atmosphérique et à la température ambiante), les valeurs de pressions absolues sont données dans le tableau suivant:

| **Débit** | **Pppe** | **Ps** | **Pe** - (L=100 m) | **Pe -** (L=200 m) |
|---|---|---|---|---|
| (ml/min) | (mb) | (mb) | (mb) | (mb) |
| 200 | 3 | 19 | 29 | 38 |
| 400 | 4 | 26 | 42 | 53 |
| 600 | 5 | 34 | 53 | 65 |
| 800 | 6 | 40 | 62 | 76 |

Il est clair que le système selon l'invention permet d'appliquer une pression absolue de quelques dizaines de millibar au niveau des moyens d'extraction, ce qui est généralement suffisant pour des hydrocarbures "lourds" C5-C8. On rappelle ici que la boucle d'échantillonnage de la présente invention est conçue et agencée sur la ligne de transport de façon à ne pas procurer de perte de charge sensible sur la ligne.

Pour que le système soit opérationnel, il faut que le temps de transit des constituants n'excède pas environ 120 secondes. Pour évaluer le temps de transit, on injecte un mélange gazeux déterminé à l'extrémité de la ligne et on détecte l'apparition de ce mélange avec un spectrographe de masse branché sur l'autre extrémité. Les valeurs suivantes on été obtenues avec une ligne de transport de 100 m de long et de diamètre 10 mm:

| **Débit** (atm.) | **Pe** | **Pppe** | **Temps de transit** |
|---|---|---|---|
| (ml/min) | (mb) | (mb) | (s) |
| 282 | 36 | 3,6 | 56 |
| 197 | 29 | 3 | 72 |
| 140 | 25 | 2,5 | 85 |
| 86 | 21 | 1,9 | 109 |
| 60 | 17 | 1,6 | 142 |

Ces mesures permettent de valider un modèle d'optimisation de fonctionnement qui prend en compte le volume de gaz extrait, y compris la vapeur d'eau, en fonction des conditions P et T qui règnent dans les moyens d'extraction. Si le volume de gaz aspiré n'est pas suffisant pour obtenir un temps de transit acceptable, on pourra, soit chauffer le fluide par l'électrode 8 (figure 1) pour obtenir plus de vapeur, soit admettre un gaz supplémentaire en contrôlant l'ouverture de la vanne 25 et le débit entrant. Pour obtenir une quantification représentative de la teneur des constituants entraînés par le fluide de forage, on fonctionnera de préférence sans apport de gaz externe par le conduit 24 (figure 1). Il est cependant possible de fonctionner avec un apport externe d'un gaz, air ou azote, dans la mesure où un calibrage du système de mesure est possible.

Les conditions d'extraction des constituants C1-C8, de transport et d'analyse, sont variables et dépendent de la nature du fluide de forage, de la nature des terrains traversés par le forage, de la vitesse de circulation du fluide de forage, de la température du fluide et de l'air ambiant. Aussi, le système selon l'invention présente l'avantage d'admettre une grande souplesse de réglage, que ce soient des conditions P et T à l'extraction, des conditions de débit et de pression pour le temps de transit et l'injection d'un échantillon dans l'analyseur chromatographique.

Pour compléter les tests sur la maquette décrite plus haut, des mesures de pressions et de temps de transit ont été effectuées avec l'incorporation de la boucle d'échantillonnage telle que décrite ci-dessus. On mesure, de plus, la perte de charge dp aux bornes de la vanne 52 (figure 3a ou 3b), c'est à dire aux bornes de la boucle d'échantillonnage 53 dans les conditions les plus défavorables, c'est à dire les vannes 56 et 57 fermées.

| **Débit** | **Pe** | **Ps** | **dp** | **Pppe** | **Temps de transit** |
|---|---|---|---|---|---|
| (Nml/min) | (mb) | (mb) | (mb) | (mb) | (s) |
| 140 | - | - | 0 | - | 85* |
| 140 | 27 | 18 | 8 (*) | 1,9 | 108 |
| 140 | 34 | 28 | 16 | 1,9 | 134 |
| 140 | 42 | 37 | 26 | 1,8 | 160 |
| 140 | 51 | 47 | 36 | 1,8 | 197 |
| 140 | 68 | 65 | >50 | 1,8 | 257 |

| | | | | | |
|---|---|---|---|---|---|
| (*) mesures effectuées sur la ligne sans boucle d'échantillonnage, pour comparaison. | | | | | |

Ces mesures montrent les réglages de la perte de charge créée par la vanne 52 afin que la boucle d'échantillonnage reçoive un débit dévié de la ligne principale, sans que l'équilibre débit/pression soit perturbé à chaque balayage de la prise d'échantillon. On a vérifié que les échantillons prélevés étaient bien représentatifs de l'effluent transporté par la ligne principale. Il est clair que la vanne 52 peut être pilotée si besoin.

## Revendications

1. Système d'extraction, d'analyse et de mesure sur des constituants entraîner par un fluide de puits au cours d'opérations de forage, **caractérisé en ce qu'**il comporte en combinaison:
• des moyens de prélèvement (4) d'un volume déterminé dudit fluide,
• des moyens d'extraction (3) sous forme vapeur desdits constituants contenus dans ledit volume de fluide comportant, un espace en dépression,
• une conduite (14) de transport des vapeurs dont une première extrémité communique avec ledit espace, la seconde extrémité étant reliée à une pompe à dépression (21),
• des moyens d'échantillonnage (27) placés dans le voisinage de la seconde extrémité comportant une boucle d'échantillonnage (53) qui permet le prélèvement d'une quantité déterminée des vapeurs circulant dans ladite conduite,
• des moyens de distribution (50) qui injectent ladite quantité dans des moyens d'analyse et de mesure (31), et
• des moyens de contrôle de la température (33) desdits moyens d'échantillonnage afin d'éviter toute condensation de ladite quantité de vapeurs.

2. Système selon la revendication 1, dans lequel ledit espace qui contient le fluide de forage prélevé est étanche via-à-vis de l'extérieur.

3. Système selon la revendication 1, dans lequel ledit espace comporte un orifice d'entrée (24) d'un gaz auxiliaire, par exemple de l'air ou de l'azote.

4. Système selon l'une des revendications précédentes, dans lequel ledits moyens d'extraction comportent des moyens de chauffage (8) dudit volume de fluide prélevé.

5. Système selon l'une des revendications précédentes, dans lequel ladite boucle d'échantillonnage (53) est montée directement en parallèle sur la ligne de transport (14).

6. Système selon l'une des revendications précédentes, dans lequel lesdits moyens de prélèvement dudit fluide de forage comprennent une pompe d'aspiration (4) et/ou une pompe de refoulement (13).

7. Système selon l'une des revendications précédentes, dans lequel ledit espace est à une pression absolue comprise entre 10 et 100 mb et l'aspiration de la pompe à dépression est comprise entre 1 et 10 mb.

8. Méthode d'extraction, d'analyse et de mesure sur des constituants entraînés par un fluide de puits au cours d'opération de forage, **caractérisée en ce que** l'on effectue les étapes suivantes:
• on prélève un volume de fluide de forage pour le placer dans un espace faisant partie de moyens d'extraction desdits constituants sous forme vapeur,
• on applique une dépression dans ledit espace par l'intermédiaire d'une conduite reliant les moyens d'analyse et de mesure aux moyens d'extraction,
• on prélève dans le voisinage des moyens d'analyse et de mesure une quantité déterminée des vapeurs circulant dans ladite conduite à l'aide d'une boucle d'échantillonage,
• on contrôle la température d'échantillonnage afin d'éviter toute condensation de ladite quantité de vapeur
• on injecte ladite quantité de vapeurs dans les moyens d'analyse et de mesure,
• on déduit la nature et/ou la quantité des constituants qui ont été vaporisés dans lesdits moyens d'extraction.

9. Méthode selon la revendication 8, dans laquelle on ajuste les conditions de dépression et/ou de température pour l'extraction sous forme vapeur en fonction des paramètres de forage, par exemple nature du fluide, débit, température ambiante, temps de transit dans la conduite.

10. Méthode selon la revendication 9, dans laquelle on règle un débit d'entrée de gaz auxiliaire dans ledit espace.

11. Méthode selon l'une des revendication 8 à 10, dans laquelle on applique une dépression comprise entre 10 et 100 mb absolu dans ledit espace d'extraction.

## Claims

1. System for extracting, analysing and measuring constituents carried along by a well fluid during drilling operations,
**characterised in that** it includes in combination:
• means for sampling (4) a determined volume of said fluid,
• means for extracting (3) in vapour form said constituents contained in said volume of fluid including a depressed space,
• a conduit (14) for transporting vapours, a first end of which is in communication with said space, with the second end connected to a depression pump (21),
• sampling means (27) placed in the vicinity of the second end including a sampling loop (53) which permits sampling of a determined quantity of the vapours travelling in said conduit,
• distribution means (50) which inject said quantity into analysis and measurement means (31), and
• means for controlling the temperature (33) of said sampling means in order to prevent any condensation of said quantity of vapours.

2. The system according to Claim 1, wherein said space which contains the sampled drilling fluid is sealed with respect to the outside.

3. The system according to Claim 1, wherein said space includes an intake port (24) for an auxiliary gas, for example air or nitrogen.

4. The system according to one of the preceding claims, wherein said extraction means include means for heating (8) said volume of sampled fluid.

5. The system according to one of the preceding claims, wherein said sampling loop (53) is directly mounted in parallel on the transport line (14).

6. The system according to one of the preceding claims, wherein said means for sampling said drilling fluid comprise a suction pump (4) and / or a recirculating pump (13).

7. The system according to one of the preceding claims, wherein said space is at an absolute pressure of between 10 and 100 mb and the suction of the depression pump is between 1 and 10 mb.

8. Method for extracting, analysing and measuring constituents carried along by a well fluid during a drilling operations,
**characterised in that** the following steps are performed:
• a volume of drilling fluid is sampled and placed in a space forming part of means for extracting said constituents in vapour form,
• a depression is applied in said space through a conduit connecting the analysis and measurement means to the extraction means,
• in the vicinity of the analysis and measurement means, a determined quantity of the vapours travelling in said conduit is sampled by means of a sampling loop,
• the sampling temperature is controlled in order to prevent any condensation of said quantity of vapours,
• said quantity of vapours is injected into the analysis and measurement means,
• the nature and / or the quantity of the constituents which have been vaporised into said extraction means are derived.

9. The method according to Claim 8, wherein the depression and / or temperature conditions for the extraction in vapour form are adjusted according to the drilling parameters, for example, the nature of the fluid, rate, ambient temperature, transit time in the conduit.

10. The method according to Claim 9, wherein an intake rate of auxiliary gas is regulated in said space.

11. The method according to one of Claims 8 to 10, wherein a depression of between 10 and 100 absolute mb is applied in said extraction space.

## Patentansprüche

1. System zur Extraktion, Analyse und Messung an Bestandteilen, die von einem Bohrlochfluid während Bohrarbeiten mitgenommen werden, **dadurch gekennzeichnet, dass** es in Kombination umfasst:
- Mittel (4) zur Entnahme eines bestimmten Volumens des Fluids,
- Mittel (3) zur Extraktion der in dem Fluidvolumen enthaltenen Bestandteile in Dampfform, umfassend einen Raum mit Unterdruck,
- eine Transportleitung (14) für die Dämpfe, deren erstes Ende mit dem Raum in Verbindung steht, wobei das zweite Ende mit einer Vakuumpumpe (21) verbunden ist,
- Probenahmemittel (27), die in der Nähe des zweiten Endes angeordnet sind und eine Probenahmeschleife (53) umfassen, die die Entnahme einer bestimmten Menge der in der Leitung zirkulierenden Dämpfe ermöglicht,
- Verteilungsmittel (50), die die Menge in Analyse- und Messmittel (31) einleiten, und
- Mittel zur Temperaturkontrolle (33) der Probenahmemittel, um jede Kondensation der Dampfmenge zu verhindern.

2. System nach Anspruch 1, bei dem der Raum, der das entnommene Bohrfluid enthält, gegen die Umgebung abgedichtet ist.

3. System nach Anspruch 1, bei dem der Raum eine Einlassöffnung (24) für ein Hilfsgas, beispielsweise Luft oder Stickstoff, umfasst.

4. System nach einem der vorhergehenden Ansprüche, bei dem die Extraktionsmittel Heizmittel (8) für das entnommene Fluidvolumen umfassen.

5. System nach einem der vorhergehenden Ansprüche, bei dem die Probenahmeschleife (53) direkt parallel auf der Transportleitung (14) montiert ist.

6. System nach einem der vorhergehenden Ansprüche, bei dem die Entnahmemittel des Bohrfluids eine Ansaugpumpe (4) und/oder eine Verdrängungspumpe (13) umfassen.

7. System nach einem der vorhergehenden Ansprüche, bei dem der Raum auf einem Absolutdruck zwischen 10 und 100 mb ist und die Absaugung der Vakuumpumpe zwischen 1 und 10 mb beträgt.

8. Methode zur Extraktion, Analyse und Messung an Bestandteilen, die von einem Bohrfluid während Bohrarbeiten mitgenommen werden, **dadurch gekennzeichnet, dass** die folgenden Schritte durchgeführt werden:
- Entnehmen eines Bohrfluidvolumens, um es in einem Raum anzuordnen, der Teil der Mittel zur Extraktion der Bestandteile in Dampfform ist,
- Anlegen eines Unterdrucks an dem Raum mit Hilfe einer Leitung, die die Analyse- und Messmittel mit den Extraktionsmitteln verbindet,
- Entnehmen einer bestimmten Menge der Dämpfe, die in der Leitung zirkulieren, mit Hilfe einer Probenahmeschleife, in der Nähe der Analyse- und Messmittel,
- Kontrollieren der Probenahmetemperatur, um jede Kondensation der Dampfmenge zu verhindern,
- Einleiten der Dampfmenge in die Analyse- und Messmittel,
- Herleiten der Beschaffenheit und/oder der Menge der Bestandteile, die in den Extraktionsmitteln verdampft wurden.

9. Methode nach Anspruch 8, bei der die Unterdruck- und/oder Temperaturbedingungen für die Extraktion in Dampfform in Abhängigkeit von den Bohrparametern, beispielsweise Beschaffenheit des Fluids, Menge, Raumtemperatur, Durchgangszeit in der Leitung, angepasst werden.

10. Methode nach Anspruch 9, bei der eine Einlassmenge an Hilfsgas in den Raum geregelt wird.

11. Methode nach einem der Ansprüche 8 bis 10, bei der ein Unterdruck zwischen 10 und 100 mb absolut in dem Extraktionsraum angelegt wird.
